# EUROPEAN PATENT APPLICATION

(11) **EP 3 066 980 A1**
(43) Date of publication of application: **14.09.2016**
(21) Application number: 14859476.5
(22) Date of filing: 10.09.2014
(51) Int. Cl.: A61B 5/0245, A61B 5/1455

(54) **LIVING BODY INFORMATION MEASUREMENT DEVICE**

(30) Priority: 08.11.2013 JP 2013231659
(71) Applicant: Konica Minolta, Inc., Tokyo 100-7015 (JP)
(72) Inventor: SHIGENAGA, Nobuaki, Tokyo 100-7015 (JP)
(74) Representative: Hoffmann Eitle
(86) International application number: PCT/JP2014/073974
(87) International publication number: WO 2015/068465

(57) **Abstract**

A living body information measurement device of the present invention includes: an insertion/removal opening through which a finger of a living body is to be inserted/removed, a receiving space for receiving the living body finger inserted through the insertion/removal opening, and a mounting main unit having a pad receiving surface facing the receiving space and being configured to receive a pad of the living body finger inserted through the insertion/removal opening, and the mounting main unit has a pair of first finger slippage blocking portions at respective side edges of the pad receiving surface in a perpendicular direction perpendicular to an insertion/removal direction in which the living body finger is to be inserted and removed, the first finger slippage blocking portions extending along the insertion/removal direction and being configured to prevent the living body finger received by the pad receiving surface from being shifted in the perpendicular direction.

## Description

### Technical Field

The present invention relates to a living body information measurement device for measuring predetermined physiological information from living bodies.

### Background Art

Various types of living body information measurement devices for measuring predetermined physiological information (living body information) from living bodies to provide conditions of the living bodies are known. For example, a pulse wave meter measures the pulse wave or the like of a living body, a pulse oximeter measures the blood oxygen saturation level or the like of a living body, and a blood glucose meter measures the blood glucose level of a living body. One example of methods for measuring living body information is a photoelectric method. A living body information measurement device based on the photoelectric method emitting and receiving light to/from a finger of a living body to measure predetermined living body information, for example. A living body information measurement device based on the photoelectric method thus has a mounting part into which the finger is to be mounted.

With such a living body information measurement device based on the photoelectric method, however, if the finger moves during emission and reception of light to/from the finger for measurement and the position of the finger is shifted from the position of the emitted/received light (measurement position), this causes errors in measured values. Thus, the positional relation between the measurement position and the finger position needs to be maintained during measurement. One example of techniques for maintaining the positional relation is used in living body information measurement device disclosed in Patent Literature 1.

A ring-type biosignal detecting device disclosed in Patent Literature 1 includes an annular mounting part to be worn on a finger, and a light emitting element and a photodetector provided on an inner face of the mounting part, in which the photodetector receives light emitted by the light emitting element toward the finger so as to detect a biosignal. The biosignal detecting device is provided with an adjustment mechanism for reducing the diameter of the inner face of the mounting part as a whole. According to Patent Literature 1, as a result of reducing the diameter of the whole inner face of the mounting part by the adjustment mechanism for reducing the diameter of the whole mounting part, the finger can be squeezed by the entire inner face of the mounting part, which can prevent the ring from turning and formation of a space between the detecting element and the finger. This enables accurate detection of a biosignal.

As described above, the biosignal detecting device disclosed in Patent Literature 1 squeezes the finger with the adjustment mechanism to stabilize the positional relation between the finger position and the positions of the light emitting element and the photodetector. With the biosignal detecting device disclosed in Patent Literature 1, however, since the adjustment mechanism squeezes the finger, the finger may be congested and the subject may feel compressed and thus stressed. In particular, measurement of the pulse wave, the blood oxygen level, and the like takes a relatively long time for monitoring (checking or observing) the condition of a subject. Thus, living body information measurement devices are also to be worn by subjects for a relatively long time, and the aforementioned drawbacks are crucial. If the squeezing of the adjustment mechanism is loosened so that the drawbacks may be avoided, the positional relation between the finger position and the positions of the light emitting element and the photodetector may become unstable.

### Citation List

### Patent Literature

Patent Literature 1: JP 11-332840 A

### Summary of Invention

The present invention has been made in view of the aforementioned circumstances, and an object thereof is to provide a living body information measurement device capable of reducing compression applied to a finger and reducing shifting of the position of the finger from the measurement position.

A living body information measurement device according to the present invention includes: an
insertion/removal opening through which a finger of a living body is to be inserted/removed, a receiving space for receiving the living body finger inserted through the insertion/removal opening, and a mounting main unit having a pad receiving surface facing the receiving space and being configured to receive a pad of the living body finger inserted through the insertion/removal opening, and the mounting main unit has a pair of first finger slippage blocking portions at respective side edges of the pad receiving surface in a perpendicular direction perpendicular to an insertion/removal direction in which the living body finger is to be inserted and removed, the first finger slippage blocking portions extending along the insertion/removal direction and being configured to prevent the living body finger received by the pad receiving surface from being shifted in the perpendicular direction. Such a living body information measurement device is capable of reducing compression applied to a finger and reducing shifting of the position of the finger from the measurement position.

The aforementioned and other objects, features and advantages of the present invention will be apparent from the detailed description below and the accompanying drawings.

### Brief Description of Drawings

Fig. 1 illustrates three views of external appearances of a living body information measurement device according to an embodiment.
Fig. 2 is a cross-sectional view of the living body information measurement device according to the embodiment taken along line I-I in Fig. 1.
Fig. 3 is a cross-sectional view of the living body information measurement device according to the embodiment including first finger slippage blocking portions (first finger slippage preventing portions) according to a first aspect taken along line II-II in Fig. 1.
Fig. 4 is a plan view of a pad receiving portion in the living body information measurement device according to the embodiment including the first finger slippage blocking portions according to the first aspect.
Fig. 5 is a cross-sectional view of the living body information measurement device according to the embodiment including first finger slippage blocking portions according to a second aspect taken along line II-II in Fig. 1.
Fig. 6 is a cross-sectional view of the living body information measurement device according to the embodiment including first finger slippage blocking portions according to a third aspect taken along line II-II in Fig. 1.
Fig. 7 is a block diagram illustrating an electrical configuration of the living body information measurement device according to the embodiment.
Fig. 8 is a perspective view of an external appearance of the living body information measurement device according to the embodiment in a state in which a finger of a human hand is inserted therein.
Fig. 9 is a partial cross-sectional view of the living body information measurement device according to the embodiment in a state in which a finger of a human hand is inserted therein.
Fig. 10 is a cross-sectional view of the living body information measurement device according to the embodiment further including second finger slippage blocking portions (second finger slippage preventing portions) taken along line II-II in Fig. 1.

### Description of Embodiments

An embodiment according to the present invention will now be described with reference to the drawings. Components denoted by the same reference numerals in the drawings are the same components, and the description thereof will not be repeated as appropriate. Herein, reference numerals without indices will be used for collective reference to components, and reference numerals with indices will be used for reference to individual components.

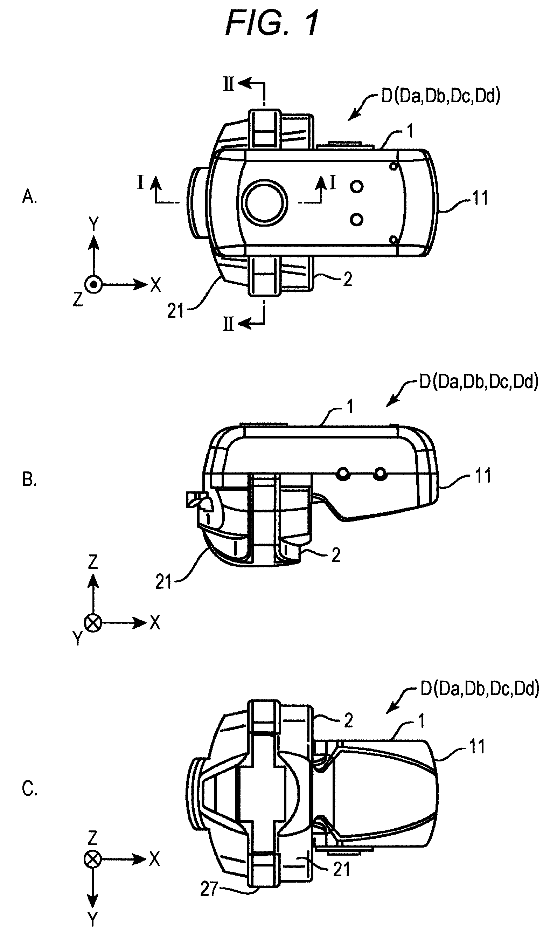

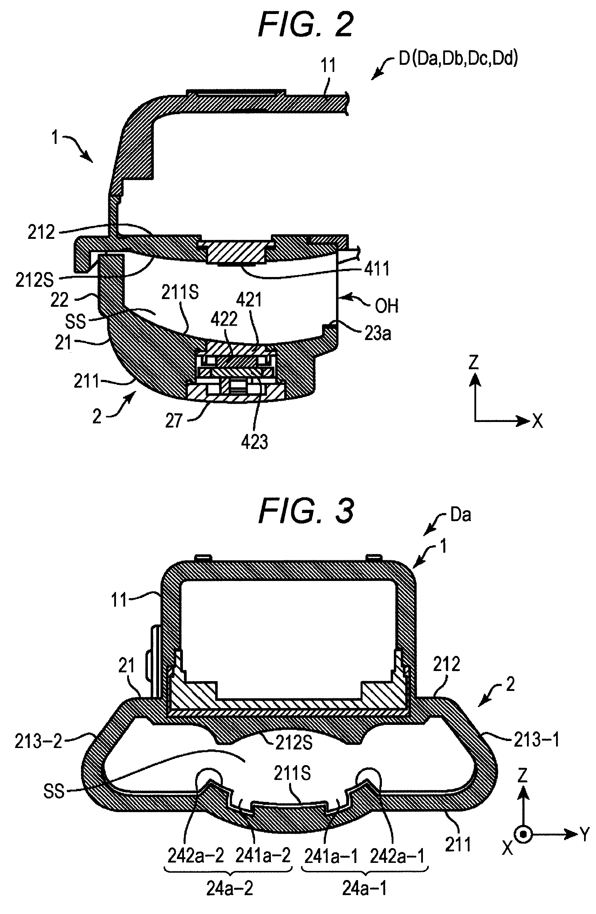

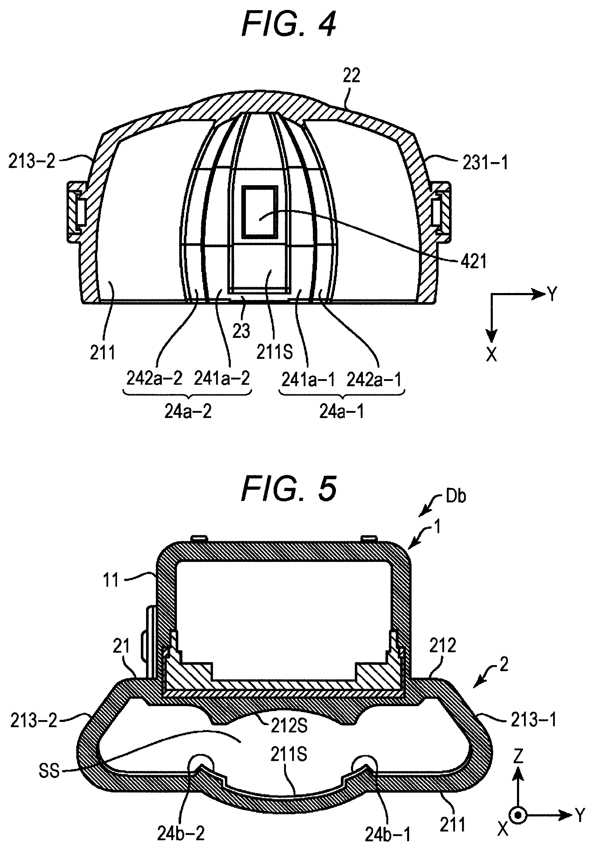

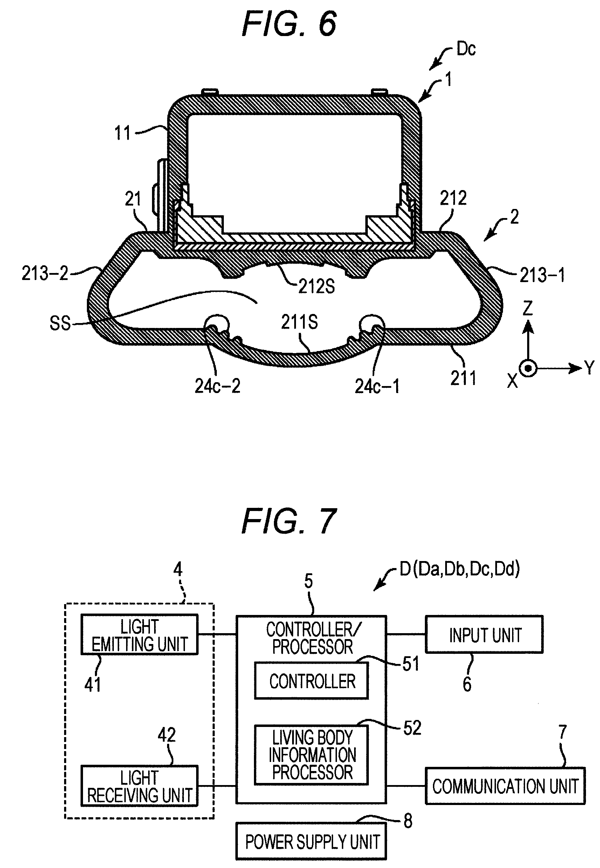

Fig. 1 illustrates three views of external appearances of a living body information measurement device according to an embodiment. Fig. 1A is a top view, Fig. 1B is a side view, and Fig. 1C is a bottom view. Fig. 2 is a cross-sectional view of the living body information measurement device according to the embodiment taken along line I-I in Fig. 1. Fig. 3 is a cross-sectional view of the living body information measurement device according to the embodiment including first finger slippage blocking portions (first finger slippage preventing portions) according to a first aspect taken along line II-II in Fig. 1. Fig. 4 is a plan view of a pad receiving portion in the living body information measurement device according to the embodiment including the first finger slippage blocking portions according to the first aspect. Fig. 5 is a cross-sectional view of the living body information measurement device according to the embodiment including first finger slippage blocking portions according to a second aspect taken along line II-II in Fig. 1. Fig. 6 is a cross-sectional view of the living body information measurement device according to the embodiment including first finger slippage blocking portions according to a third aspect taken along line II-II in Fig. 1. Fig. 7 is a block diagram illustrating an electrical configuration of the living body information measurement device according to the embodiment.

The living body information measurement device according to the embodiment is a device worn on a part of a living body to measure predetermined physiological information (living body information) of the living body. More specifically, the living body information measurement device is worn on a finger (a finger of a hand, for example) of a living body, and is configured to emit predetermined measurement light to the finger of the living body, receive detection light based on the measurement light and coming from the inside of the finger, and obtain predetermined living body information on the basis of the light reception result. Examples of the predetermined living body information include the pulse wave, the pulse rate, the pulse period, the blood oxygen saturation level, and the blood glucose level of the living body.

Such a living body information measurement device D (Da, Db, Dc) includes a main unit 1 and a mounting part 2 into which a finger of a living body is to be inserted as illustrated in Figs. 1 to 6, for example. In Figs. 1 to 6, an XYZ orthogonal coordinate system in which the longitudinal direction of the living body information measurement device D is an X direction is provided, which is used as appropriate for description of the living body information measurement device D.

The main unit 1 is configured to process a light reception result obtained by emitting and receiving light to/from a finger inserted in the mounting part 2, and includes a box-like housing 11 having a substantially rectangular parallelepiped shape and electrical components 4 to 8 illustrated in Fig. 7. The housing 11 is formed of a material, such as a resin material including plastic, capable of protecting the components 4 to 8 during use, transportation, storage, and the like of the living body information measurement device D through molding using a mold, for example.

Description of the electrical configuration of the living body information measurement device D will be given here. The living body information measurement device D includes a light emitting/receiving unit 4, a controller/processor 5, an input unit 6, a communication unit 7, and a power supply unit 8 as illustrated in Fig. 7, for example.

The light emitting/receiving unit 4 is connected to the controller/processor 5, and is configured to emit predetermined measurement light to a finger of a living body and receive detection light based on the measurement light and coming from the inside of the finger for use in obtaining predetermined living body information. More specifically, the light emitting/receiving unit 4 includes a light emitting unit 41 configured to emit measurement light to the finger according to control performed by the controller/processor 5, and a light receiving unit 42 configured to received detection light and output a light reception result (photoelectric pulse wave signal) to the controller/processor 5. The detection light is obtained as a result of the measurement light passing through the finger or as a result of the measurement light being reflected in the finger. Thus, the light emitting unit 41 and the light receiving unit 42 are disposed in a relation suitable for using the transmitted detection light (such as being disposed opposite to each other or being apposed with a space therebetween) or in a relation suitable for using the reflected detection light (such as being apposed adjacent to each other). the living body information measurement device D according to the present embodiment, as will be described later, the light emitting unit 41 and the light receiving unit 42 are disposed to face each other with a predetermined space therebetween (disposed opposite to each other) so that the transmitted detection light can be used. The measurement light is light having a wavelength appropriately selected according to the type of living body information to be measured by the living body information measurement device D. For example, the measurement light is red light or the like, for example, in a case where the pulse wave, the pulse rate, the pulse period, and the like are to be measured as the living body information. For example, the measurement light includes a plurality of types of light having different absorbances such as two types of light, which are red light and infrared light, in a case where the blood oxygen level, the blood glucose level, and the like are to be measured as the living body information. The light emitting unit 41, for example, includes an irradiation optical system, including a power supply such as a light emitting diode (LED) and a peripheral circuit thereof such as a drive circuit, for example. The light receiving unit 42 includes a light receiving optical system, including a photoelectric conversion element such as a silicon photodiode and a peripheral circuit thereof such as a current/voltage conversion circuit, for example.

The controller/processor 5 is a circuit for controlling the respective components of the living body information measurement device D according to the functions of the respective components to obtain predetermined living body information on the basis of the light reception result of the light receiving unit 42 in the light emitting/receiving unit 4, and includes a microcomputer including a central processing unit (CPU), a memory, and peripheral circuits thereof, for example. The controller/processor 5 includes a controller 51 and a living body information processor 52 functionally implemented by executing programs. The controller 51 is for controlling the respective components of the living body information measurement device D according to the functions of the respective components. The living body information processor 52 is for obtaining predetermined living body information on the basis of a light reception result (photoelectric pulse wave signal) of the light receiving unit 42 in the light emitting/receiving unit 4. For a method for computing the living body information, a known common practice disclosed in JP H1-153139 A (an application claiming priority of from GB 8719333 filed on August 14, 1987) or JP 2007-167184 A, for example.

For one example, in JP H1-153139 A, the oxygen saturation level is roughly calculated by (AC1/DC1) / (AC2/DC2), where DC1 and DC2 represent DC components of first and second signals, respectively, obtained by a photodetector when first light having a wavelength of 660 nm and second light having a wavelength of 940 nm are emitted to a living body, and AC1 and AC2 represent AC components thereof. For another example, in JP 2007-167184 A, the oxygen saturation level is roughly obtained by using differences between the absorbances of oxygenated hemoglobin and hemoglobin in the case of red light having a wavelength of 660 nm and in the case of infrared light having a wavelength of 815 nm. Specifically, while the difference in absorption coefficient between oxygenated hemoglobin and hemoglobin is the greatest in the case of red light having a wavelength of 660 nm, the difference in absorption coefficient between oxygenated hemoglobin and hemoglobin is equal in the case of infrared light having a wavelength of 815 nm. Thus, the amount of transmitted red light is larger as the amount of hemoglobin is larger, while the amount of transmitted infrared light remains unchanged even when the ratio of oxygenated hemoglobin to hemoglobin has changed. In this manner, the ratio of the amounts of transmitted red light to transmitted infrared light is obtained so that the oxygen saturation level can be obtained. As described above, the living body information is obtained by a known common practice.

The input unit 6 is a circuit connected to the controller/processor 5, and used for inputting instructions such as on/off and start of measurement, and includes a button switch, for example. The communication unit 7 is a circuit connected to the controller/processor 5 and used for transmitting communication signals to an external device in a wireless manner, for example. Transmission of the living body information obtained by the controller/processor 5 through the communication unit 7 eliminates the need for mounting a configuration for analyzing and saving the living body information on the living body information measurement device D, which results in omission of a display unit, miniaturization, power saving and lower cost. Note that the communication unit 7 may transmit the light reception result (photoelectric pulse wave signal) obtained by the light receiving unit 42 of the light emitting/receiving unit 4 without any change to an external device. This allows the living body information processor 52 of the controller/processor 5 to be omitted. The power supply unit 8 is a circuit for supplying power to the respective components requiring power, such as the light emitting unit 41, the controller/processor 5 and the communication unit 7, for example, in the living body information measurement device D. The power supply unit 8 includes a battery such as a secondary battery and a peripheral circuit thereof.

A photodetector 422, which will be described later, of the light receiving unit 42 is contained in the mounting part 2, and the other components, which are the light emitting/receiving unit 4, the controller/processor 5, the input unit 6, the communication unit 7, and the power supply unit 8, are contained in the housing 11.

The description refers back to Fig. 1 to Fig. 6. The mounting part 2 is a member into which a finger of a living body (such as a finger of a human hand) is inserted for measurement of predetermined living body information, and is integrally attached and coupled to the main unit 1. More specifically, in the present embodiment, the mounting part 2 includes a mounting main unit 21, a stopper portion 22, a first finger retaining portion 23, and first finger slippage blocking portions 24, for example.

The mounting main unit 21 is a member having an insertion/removal opening OH through which a finger of a living body is inserted and removed, a receiving space SS in which the finger of the living body inserted through the insertion/removal opening OH is to be received, and a pad receiving surface 211S facing the receiving space SS and being configured to receive the pad (the inner side of the finger tip, the fingerprint portion, or the surface opposite to the nail) of the living body finger inserted through the insertion/removal opening OH. In the present embodiment, the mounting main unit 21 also has a back receiving surface 212S facing the receiving space SS and being configured to receive the back (the outer side of the finger tip, or the nail-side surface) of the living body finger inserted through the insertion/removal opening OH. For example, in the present embodiment, the mounting main unit 21 of the mounting part 2 includes a pad receiving portion 211, a back receiving portion 212, and a pair of first and second connecting portions 213-1 and 213-2.

The pad receiving portion 211 is a plate-like member of a substantially rectangular shape having the pad receiving surface 211S configured to receive the pad of the living body finger on one side thereof when the living body finger is inserted into the receiving space SS through the insertion/removal opening OH. The back receiving portion 212 is a plate-like member of a substantially rectangular shape having the back receiving surface 212S configured to receive the back of the living body finger on one side thereof when the living body finger is inserted into the receiving space SS through the insertion/removal opening OH. The pair of connecting portions 213-1 and 213-2 are plate-like members of a substantially rectangular shape and connects the pad receiving portion 211 and the back receiving portion 212 with each other.

More specifically, the pad receiving portion 211 and the back receiving portion 212 are disposed to face each other with a predetermined distance between the pad receiving surface 211S and the back receiving surface 212S to form the receiving space SS, one end side in the transverse direction (Z direction) (i.e., the end side in +Z direction) of the first connecting portion 213-1 is connected with one end side in the longitudinal direction (Y direction) (i.e., the end side in +Y direction of the back receiving portion 212, and the other end side in the transverse direction (Z direction) (i.e., the end side in -Z direction) of the first connecting portion 213-1 is connected with the other end side in the longitudinal direction (Y direction) (i.e., the end side in +Y direction) of the pad receiving portion 211. In addition, one end side in the transverse direction (Z direction) (i.e., the end side in +Z direction) of the second connecting portion 213-2 is connected with the other end side in the longitudinal direction (Y direction) (i.e., the end side in -Y direction) of the back receiving portion 212, and the other end side in the transverse direction (Z direction) (i.e., the end side in -Z direction) of the second connecting portion 213-1 is connected with the other end side in the longitudinal direction (Y direction) (i.e., the end side in -Y direction) of the pad receiving portion 211. In this manner, the pad receiving portion 211, the first connecting portion 213-1, the back receiving portion 212, the second connecting portion 213-2, and the pad receiving portion 211 are sequentially connected in this manner in the circumferential direction (about the X axis), and the pad receiving portion 211, the first connecting portion 213-1, the back receiving portion 212, and the second connecting portion 213-2 forms a hollow columnar member (cylindrical member) of a hollow columnar shape (cylindrical shape). One end opening of the hollow columnar member constitutes the insertion/removal opening OH through which a finger of a living body is to be inserted/removed, and the internal space of the hollow columnar member constitutes the receiving space SS to receive the living body finger inserted through the insertion/removal opening (the one end opening) OH. Thus, the pad receiving surface 211S of the pad receiving portion 211 faces toward the +Z direction and the receiving space SS, and the back receiving surface 2121S of the back receiving portion 212 faces toward the -Z direction and the receiving space SS.

Such pad receiving portion 211, back receiving portion 212, and first and second connecting portions 213-1 and 213-2 are formed integrally through molding using a mold, for example, contain an elastic material exhibiting elastic force for holding a finger, and constitute the mounting main unit 21. Examples of the elastic material includes a polymeric material such as rubber, and a spring. For example, substantially the whole mounting main unit 21 (the pad receiving portion 211, the back receiving portion 212, and the first and second connecting portions 213-1 and 213-2) may be made of plastic such as rubber having elasticity, or alternatively be made of plastic containing a leaf spring of a substantially U-shape therein. The mounting main unit 21 is also made so that the distance between the pad receiving surface 211S of the pad receiving portion 211 and the back receiving surface 212S of the back receiving portion 212 is smaller than the thickness of the finger to be received by the receiving space SS and that the elastic force acts in the closing direction of the receiving space along the Z direction.

The inner surface of the back receiving portion 212 is gradually increased in height radially inward (-Z direction) of the hollow columnar member, from substantially the center position in the longitudinal direction (Y direction) toward both directions along the longitudinal direction (+Y direction and -Y direction). In this manner, a concave portion curving radially outward (+Z direction) of the hollow columnar member is formed at substantially the center in the longitudinal direction (Y direction) of the back receiving portion 212, so that the concave portion has a curving surface substantially corresponding to the back of a living body finger. The curving surface of the concave portion constitutes the back receiving surface 212S. In addition, a through-hole of a predetermined shape extending and being open in the thickness direction (Z direction) of the back receiving portion 212 is formed at substantially the center of the back receiving surface 212S of the back receiving portion 212, and the through-hole is provided with a light emitting window portion 411. The light emitting window portion 411 is a plate-like member being fittable into the through-hole and formed of a material transmitting at least light having the wavelength of the measurement light, one face of the light emitting window portion 411 faces the receiving space SS and is flush with the back receiving surface 212S, and the other face thereof opposite to the aforementioned face faces a light emitting surface of a light emitting element, which is not illustrated, of the light emitting unit 41. As a result of forming the through-hole in the back receiving surface 212S and placing the light emitting window portion 411 as described above, the back receiving surface 212S includes a measurement region surface (the surface of the light emitting window portion 411 in the present embodiment) to which the measurement light is emitted for measurement of predetermined living body information.

A concave portion curving radially outward of the hollow columnar member is formed at substantially the center in the longitudinal direction (Y direction) of the pad receiving portion 211. The concave portion is curved both in the longitudinal direction (Y direction) and the transverse direction (X direction) perpendicular thereto so that the curving surface substantially corresponds to the external shape of a finger of a living body, where the curing surface constitutes the pad receiving surface 211S. In addition, a through-hole of a predetermined shape extending and being open in the thickness direction (Z direction) of the pad receiving portion 211 is formed at substantially the center of the pad receiving surface 211S of the pad receiving portion 211, and the through-hole is provided with a light receiving window portion 421, the photodetector 422, a photodetector substrate 423, and a fastening band portion 27 stacked in this order from the radially inside to radially outward of the hollow columnar member. The light receiving window portion 421 is a plate-like member being fittable into the through-hole and formed of a material transmitting at least light having the wavelength of the detection light, one face of the light receiving window portion 412 faces the receiving space and is flush with the pad receiving surface 211S, and the other face thereof opposite to the aforementioned face faces the light receiving surface of the photodetector 422. As a result of forming the through-hole in the pad receiving surface 211S and placing the light receiving window portion 421 as described above, the pad receiving surface 211S includes a measurement region surface (the surface of the light receiving window portion 412 in the present embodiment) for receiving the detection light for measurement of predetermined living body information. The photodetector 422 is the photoelectric conversion element of the light receiving unit 42 described above, and the photodetector substrate 423 is a support member for supporting the photodetector 422. The fastening band portion 27 is a band-like member having a portion on one side of the center thereof extending along the circumferences of parts of the pad receiving portion 211, the first connecting portion 213-1, the back receiving portion 212, the end of the portion on one side being connected and fixed to one side surface of the housing 11 of the main unit 1, and a portion on the other side of the center thereof extending along the circumferences of parts of the pad receiving portion 211, the second connecting portion 213-2, and the back receiving portion 212, the end of the portion on the other side being connected and fixed to the other side surface of the housing 11 of the main unit 1, as illustrated in Fig. 1. In this manner, the fastening band portion 27 holds the light receiving window portion 421, the photodetector 422, and the photodetector substrate 423 inside the through-hole, and thus prevents the light receiving window portion 421, the photodetector 422, and the photodetector substrate 423 from falling off the through-hole.

The stopper portion 22 is a member for stopping a finger of a living body, and positioned in an opening (an opening opposite to the insertion/removal opening OH) at the other end of the hollow columnar member. More specifically, the stopper portion 22 is a protrusion extending for a predetermined length radially inward of the hollow columnar member (+Z direction) from the other end (the end in -X direction) of the pad receiving portion 211 in an opening at the other end of the hollow columnar member. A living body finger inserted into the receiving space SS through the insertion/removal opening OH of the mounting main unit 21 comes in contact with the stopper portion 22, and the stopper portion 22 thus prevents the living body finger from deviating from the receiving space SS and readily and properly positions the living body finger to the position to/from which light is to be emitted and received for measurement of predetermined living body information.

The first finger retaining portion (first finger slippage preventing portion) 23 is a member disposed between the location of the light emitting/receiving unit 4 and the open position of the insertion/removal opening OH, to prevent a living body finger inserted into the receiving space SS from slipping off from the receiving space SS. More specifically, in the present embodiment, the first finger retaining portion 23 is positioned on the pad receiving surface 211S between the location of the measurement region surface (the surface of the light receiving window portion 412 in the present embodiment) for receiving the detection light and the open position of the insertion/removal opening OH. The first finger retaining portion 23 is a projection protruding toward the inside of the receiving space SS, having a predetermined height from the pad receiving surface 211S, and intersecting with an insertion/removal direction in which a finger of a living body is inserted/removed. More specifically, the first finger retaining portion 23 is a projection, which is positioned at one end (an end on the side of the insertion/removal opening OH) of the hollow columnar member, extends for a predetermined length in the longitudinal direction (Y direction), and is higher than the pad receiving surface 211S by a predetermined height radially inward of the hollow columnar member (+Z direction). In the present embodiment, at one end opening (on the side of the insertion/removal opening OH) of the hollow columnar member, one end (the end in +X direction) of the pad receiving portion 211 extends for a predetermined length radially inward of the hollow columnar member (+Z direction) to form the first finger retaining portion 23. Thus, the first finger retaining portion 23, which is a projection, is positioned to be perpendicular to the insertion/removal direction. The predetermined height (the length in the Z direction) is several mm, preferably about 1 to 2 mm, for example, in terms of effectively retaining a finger. The predetermined width (the length in the X direction) is submillimeter to several mm, preferably about 0.5 to 2 mm, for example, in terms of effectively retaining a finger. Furthermore, the predetermined length (the length in the Y direction) is about several mm to a dozen mm, preferably about 5 to 10 mm, for example, in terms of effectively retaining a finger. In the present embodiment, the predetermined length is equal to the length in the longitudinal direction (Y direction) of the pad receiving surface 211S as illustrated in Fig. 4.

The first finger slippage blocking portions 24 (24-1, 24-2) constitute a member, which extends along the insertion/removal direction (the transverse direction of the pad receiving portion 211; the X direction) in which a finger of a living body is inserted/removed, for preventing the living body finger received by the pad receiving surface 211S from shifting in the perpendicular direction (Y direction) perpendicular to the insertion/removal direction, and the paired first finger slippage blocking portions 24 are provided on respective side edges of the pad receiving surface 211S in the perpendicular direction.

For such first finger slippage blocking portions 24, configurations according to first to third aspects are suitably used, for example. Finger slippage blocking portions 24a (24a-1, 24a-2) of the first aspect and living body information measurement device Da having the same are illustrated in Figs. 3 and 4, finger slippage blocking portions 24b (24b-1, 24b-2) of the second aspect and a living body information measurement device Db having the same are illustrated in Fig. 5, and finger slippage blocking portions 24c (24c-1, 24c-2) of the third aspect and a living body information measurement device Dc having the same are illustrated in Fig. 7.

As illustrated in Figs. 3 and 4, the pair of first finger slippage blocking portions 24a of the first aspect are a pair of grooves/projections 24a-1, 24a-2 having shapes extending along the insertion/removal direction (X direction), which are combinations of grooves 241a (241a-1, 241a-2) recessed by a predetermined depth in the pad receiving surface 211S outward of the receiving space SS and projections 242a (242a-1, 242a-2) positioned adjacent to the grooves 241a (241a-1, 241a-2), respectively, on the outer sides thereof in the perpendicular direction (outward in the Y direction) and protruding toward the inside of the receiving space SS with a predetermined height from the pad receiving surface 211S, respectively. For example, in the present embodiment, at each of the outer sides of both side edges of the pad receiving surface 211S, the pad receiving portion 211 is formed to extend along the insertion/removal direction (X direction) while being recessed with a predetermined width and with a predetermined depth outward of the receiving space SS from the pad receiving surface 211S, continue outward in the perpendicular direction (outward in the Y direction), and extend along the insertion/removal direction (X direction) while protruding with a predetermined width and with a predetermined height from the bottom of the recess toward the inside of the receiving space SS, which forms the pair of first finger slippage blocking portions 24a of the first aspect constituted by the pair of grooves/projections 24a-1 and 24a-2. The predetermined depth (the length in the Z direction) of the grooves 241a in the pad receiving surface 211S is submillimeter to several mm, preferably about 0.5 to 1.5 mm, for example, in terms of effectively preventing finger slippage. The predetermined width (the length in the Y direction) of the grooves 241a is several mm, preferably about 2 to 4 mm, for example, in terms of effectively preventing finger slippage. Furthermore, the predetermined height (the length in the Z direction) of the projections 242a from the pad receiving surface 211S is submillimeter to several m, preferably about 0.5 to 1.5 mm, for example, in terms of effectively preventing finger slippage. The predetermined width (the length in the Y direction) of the projections 242a is several mm, preferably about 2 to 4 mm, for example, in terms of effectively preventing finger slippage.

As illustrated in Fig 5, a pair of first finger slippage blocking portions 24b according to the second aspect are a pair of projections 24b-1 and 24b-2 having shapes extending along the insertion/removal direction (X direction) and protruding toward the inside of the receiving space SS with a predetermined height from the pad receiving surface 211S. For example, in the present embodiment, at each of the outer sides of both side edges of the pad receiving surface 211S, the pad receiving portion 211 is formed to extend along the insertion/removal direction (X direction) while protruding with a predetermined width and with a predetermined height from the pad receiving surface 211S toward the inside of the receiving space SS, which forms the pair of first finger slippage blocking portions 24b of the second aspect constituted by the pair of projections 24b-1 and 24b-2. The predetermined height (the length in the Z direction) is submillimeter to several mm, preferably about 0. 5 to 1. 5 mm, for example, in terms of effectively preventing finger slippage. The predetermined width (the length in the Y direction) is several mm, preferably about 2 to 4 mm, for example, in terms of effectively preventing finger slippage.

As illustrated in Fig. 6, a pair of first finger slippage blocking portions 24c according to the third aspect are a pair of roughened surfaces obtained by surface roughening. For example, in the present embodiment, at each of the outer sides of both side edges of the pad receiving surface 211S, the pair of first finger slippage blocking portions 24c of the third aspect are a pair of rough surface layers obtained by roughening a rectangular region, which is elongated in the insertion/removal direction (X direction), of the surface of the pad receiving portion 211 in the Z direction. The rough surface layers are formed by transferring a rough surface pattern formed on a mold, for example. The length in the transverse direction (Y direction) of the rectangular shape of each of the first finger slippage blocking portions 24c is about several mm, preferably about 4 to 8 mm, for example, in terms of effectively preventing finger slippage. The surface roughness is a ten-point average roughness Rz of about 0.03 mm to 0.3 mm, for example.

In addition, the length of the pairs of first finger slippage blocking portions 24a to 24c according to the first to third aspects in substantially the X direction is a length over the whole side edges of the pad receiving surface 211S (see Fig. 4 for the first aspect) ; alternatively, the length may be a length over part of the side edges of the pad receiving surface 211S. For example, the length may be a length including the whole side edges of the light receiving window portion 421 and over part of the side edges of the pad receiving surface 211S. The pairs of first finger slippage blocking portions 24a to 24c having such a length are positioned at the outer sides of both side edges of the pad receiving surface 211S corresponding to both side edges of the light receiving window portion 421. Although the pairs of first finger slippage blocking portions 24a to 24c according to the first to third aspects are continuous in substantially the X direction in the description above, the pairs of first finger slippage blocking portions 24a to 24c may alternatively be intermittent like a broken line.

The main unit 1 and the mounting part 2 as described above are such that the length of the main unit 1 in the longitudinal direction (X direction) is longer than the length of the mounting part 2 in the X direction, which is twice the length of the mounting part 2 in the X direction or longer. The mounting part 2 is integrally connected with the main unit 1 by a predetermined connection means with its center position in the X direction shifted toward the other end (the end in -X direction) from the center position in the X direction of the main unit 1. Examples of the connection means include integrally molding, fastening with a screw or the like, adhesion with adhesive, and fitting of a recess and a protrusion. As a result of the connection with the length relations and positional relations as described above, even when the living body information measurement device D is worn on a finger, a movement of bending the finger at a joint is less interfered with, which reduces the burden on the subject even when the device is worn on a finger for a relatively long time.

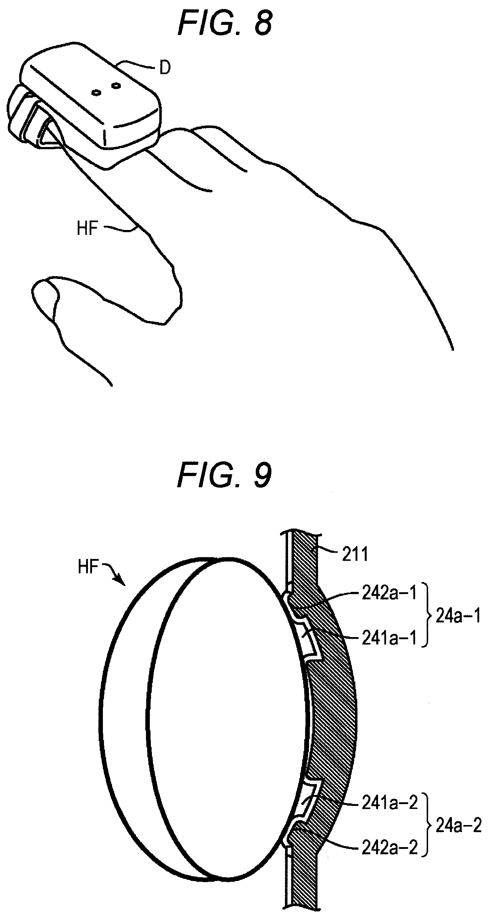

Fig. 8 is a perspective view of an external appearance of the living body information measurement device according to the embodiment in a state in which a finger of a human hand is inserted therein. Fig. 9 is a partial cross-sectional view of the living body information measurement device according to the embodiment in a state in which a finger of a human hand is inserted therein.

For measurement of living body information using such a living body information measurement device D (Da to Dc), first, in a state where the living body information measurement device D is not worn on a finger of a living body, the elastic force for holding the finger of the living body to be inserted into the receiving space SS through the insertion/removal opening OH is exhibited by the elastic material of the mounting main unit 21, and the insertion/removal opening OH and the receiving space SS are elastically deformed in the closing direction along the Z direction, as illustrated in Figs. 3, 5, and 6, for example. In this case, the distance from the pad receiving surface 211S to the back receiving surface 212S in the elastically-deformed receiving space SS is shorter (narrower) than the thickness of the finger of the living body to be measured.

Then, when the living body information measurement device D is worn on the living body finger HF, the insertion/removal opening OH and the receiving space SS are elastically deformed to be expanded in the -Z direction against the elastic force exhibited by the elastic material of the mounting main unit 21, and the living body finger HF is inserted into the receiving space SS through the insertion/removal opening OH in the X direction as illustrated in Fig. 8. As a result, in the state in which the living body information measurement device D is worn on the living body finger HF, the elastic force exhibited by the elastic material of the mounting main unit 21 acts to deform the insertion/removal opening OH and the receiving space SS in the closing direction along the Z direction, which makes the living body finger HF be held by the mounting main unit 21. In this case, for example, the living body finger HF may be placed in the receiving space SS so that a region from the tip to the first joint of the living body finger HF inserted into the receiving space SS through the insertion/removal opening OH is irradiated with measurement light emitted by the light emitting unit 41 of the light emitting/receiving unit 4 and that detection light caused by the measurement light is received by the light receiving unit 42 of the light emitting/receiving unit 4 through the living body finger HF.

In addition, in the living body information measurement device D (Da to Dc) according to the present embodiment, a pair of first finger slippage blocking portions 24 (24a to 24c) extending in the insertion/removal direction are arranged on both side edges of the pad receiving surface 211S. Thus, the living body finger placed on the pad receiving surface 211S and received by the pad receiving surface 211S is prevented from turning about the insertion/removal direction (turning about the X axis) or being shifted in the perpendicular direction (shifted in the Y direction) by the pair of first finger slippage blocking portions 24. Consequently, the living body information measurement device D according to the present embodiment is capable of reducing compression applied to a finger and reducing shifting of the position of the finger from the measurement position.

Furthermore, the living body information measurement device D according to the present embodiment can achieve the first finger slippage blocking portions relatively easily with one of the pair of projections 24a of the first aspect, the pair of grooves/projections 24b of the second aspect, and the pair of roughened surfaces 24c of the third aspect. In a case where the pair of first finger slippage blocking portions 24 are constituted by the pair of projections 24a, a living body finger placed on the pad receiving surface 211S and received by the pad receiving surface 211S hits either one of the pair of projections 24a when the living body finger attempts to turn about the insertion/removal direction or shift its position in the perpendicular direction, the turning about the insertion/removal direction and the shift in position in the perpendicular direction are thus blocked, and the finger position is more stabilized. In a case where the pair of first finger slippage blocking portions 24 are constituted by the pair of grooves/projections 24b, a living body finger placed on the pad receiving surface 211S and received by the pad receiving surface 211S hits the projection 242b of at least one of the pair of grooves/projections 24b when the living body finger attempts to turn about the insertion/removal direction or shift its position in the perpendicular direction, the turning about the insertion/removal direction and the shift in position in the perpendicular direction are thus blocked, and the finger position is more stabilized. In addition, the grooves 241b of the grooves/projections 24b allow high breathablitity to be achieved, and the grooves 241b also reduce compression of the finger. In particular, even in cases where the living body information measurement device D is worn for a relatively long time for monitoring (checking) the condition of the subject, the finger is held under a better condition. In a case where the pair of first finger slippage blocking portions 24 are constituted by the pair of roughened surfaces 24c, a living body finger placed on the pad receiving surface 211S and received by the pad receiving surface 211S hits either one of the pair of roughened surfaces 24c when the living body finger attempts to turn about the insertion/removal direction or shift its position in the perpendicular direction, the turning about the insertion/removal direction and the shift in position in the perpendicular direction are thus blocked, and the finger position is more stabilized.

In the embodiments described above (including the first finger slippage blocking portions 24a to 24c of the first to third aspects; the same shall apply hereinafter), the living body information measurement device D (Da to Dc) may further include second finger slippage blocking portions.

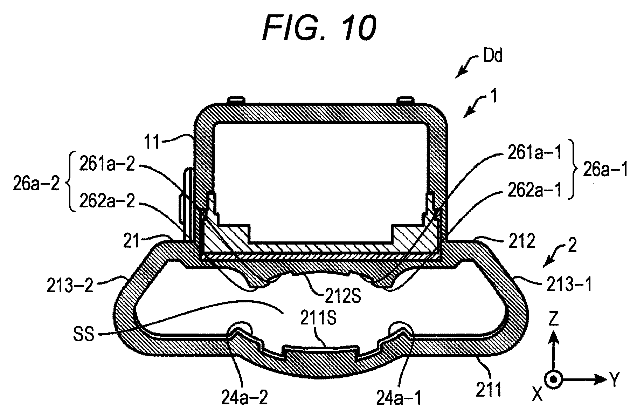

Fig. 10 is a cross-sectional view of the living body information measurement device according to the embodiment further including second finger slippage blocking portions (second finger slippage preventing portions) taken along line I-I in Fig. 1.

The second finger slippage blocking portions (second finger slippage preventing portions) 26 constitute a member, which extends along the insertion/removal direction (the transverse direction of the back receiving portion 212; the X direction) in which a finger of a living body is inserted/removed, for preventing the living body finger received by the pad receiving surface 211S from shifting in the perpendicular direction (Y direction), and the paired second finger slippage blocking portions 26 are provided on the respective side edges of the back receiving surface 212S in the perpendicular direction.

For such second finger slippage blocking portions 26, the configurations according to first to third aspects are suitably used similarly to the first finger slippage blocking portions 24. Second finger slippage blocking portions 26a constituted by a pair of grooves/projections is taken here as a representative for explanation. As illustrated in Fig. 10, the second finger slippage blocking portions 26a of this aspect are a pair of grooves/projections 26a-1, 26a-2 having shapes extending along the insertion/removal direction (X direction), which are combinations of grooves 261a (261a-1, 261a-2) recessed by a predetermined depth in the back receiving surface 212S outward of the receiving space SS and projections 262a (262a-1, 262a-2) positioned adjacent to the grooves 261a (261a-1, 261a-2), respectively, on the outer sides thereof in the perpendicular direction (outward in the Y direction) and protruding toward the inside of the receiving space SS with a predetermined height from the back receiving surface 212S, respectively. For example, in the present embodiment, at each of the outer sides of both side edges of the back receiving surface 212S, the back receiving portion 212 is formed to extend along the insertion/removal direction (X direction) while being recessed with a predetermined width and with a predetermined depth outward of the receiving space SS from the back receiving surface 212S, continue outward in the perpendicular direction (outward in the Y direction), and extend along the insertion/removal direction (X direction) while protruding with a predetermined width and with a predetermined height from the bottom of the recess toward the inside of the receiving space SS, which forms the pair of second finger slippage blocking portions 26a constituted by the pair of grooves/projections 26a-1 and 26a-2. The predetermined depth (the length in the Z direction) of the grooves 261a in the back receiving surface 212S is submillimeter to several mm, preferably about 0.5 to 1.5 mm, for example, in terms of effectively preventing finger slippage. The predetermined width (the length in the Y direction) of the grooves 261a is several mm, preferably about 2 to 4 mm, for example, in terms of effectively preventing finger slippage. Furthermore, the predetermined height (the length in the Z direction) of the projections 262a from the back receiving surface 212S is submillimeter to several m, preferably about 0.5 to 1.5 mm, for example, in terms of effectively preventing finger slippage. The predetermined width (the length in the Y direction) of the projections 262a is several mm, preferably about 2 to 4 mm, for example, in terms of effectively preventing finger slippage.

In addition, the length of the pairs of second finger slippage blocking portions 26a in substantially the X direction may be a length over the whole side edges of the back receiving surface 212S, or may alternatively be a length over part of the side edges of the back receiving surface 212S. For example, the length may be a length including the whole side edges of the light emitting window portion and over part of the side edges of the back receiving surface 212S. The pairs of second finger slippage blocking portions 26a having such a length are positioned at the outer sides of respective side edges of the back receiving surface 212S corresponding to respective side edges of the light emitting window portion. The pairs of second finger slippage blocking portions 24a may be continuous in substantially the X direction, or may alternatively be intermittent like a broken line.

In such a living body information measurement device Dd, the pair of second finger slippage blocking portions 26 extending in the insertion/removal direction are provided at respective side edges of the back receiving surface 212S, in addition to the first finger slippage blocking portions 24 (24a to 24c) at the pad receiving surface 211S. Thus, the living body finger placed on the pad receiving surface 211S and received by the pad receiving surface 211S is further prevented from turning about the insertion/removal direction (turning about the X axis) or being shifted in the perpendicular direction (shifted in the Y direction) by the respective pairs of first and second finger slippage blocking portions 26. Consequently, such a living body information measurement device Dd is capable of further reducing compression applied to a finger and further reducing shifting of the position of the finger from the measurement position.

Furthermore, although the living body information measurement device D (Da to Dd) includes the light emitting window portion 411 through which the measurement light is to be emitted is formed on the back receiving portion 22 and the light receiving window portion 412 for receiving the detection light is formed on the pad receiving portion 21 for measurement of predetermined living body information in the embodiment described above, these may be reversed. Specifically, the light emitting window portion 411 through which the measurement light is to be emitted may be formed on the pad receiving portion 21, and the light receiving window portion 412 for receiving detection light may be formed on the back receiving portion 22. Thus, the pad receiving surface 211S (or the back receiving surface 212S) contains one measurement region surface of an irradiated surface (the surface of the light emitting window portion 411 in the example above) to be irradiated with measurement light and a light receiving surface (the surface of the light receiving window portion 412 in the example above) to receive detection light.

Furthermore, in the living body information measurement device D (Da to Dd) of the embodiment described above, the mounting main unit 21 is the hollow columnar member including the pad receiving portion 211, the back receiving portion 212, and pairs of first and second connecting portions 213-1 and 213-2, and having an annular cross section, but the mounting main unit 21 is not limited thereto. The mounting main unit 21 may be in any form that can be mounted on a finger of a living body. For example, the mounting main unit 21 may be a hollow columnar member including the pad receiving portion 211, the back receiving portion 212, and a connecting portion 213-1 (or a connecting portion 213-2) connecting the pad receiving portion 211 and the back receiving portion 212 to each other at one end side thereof, and having a substantially C-shaped (substantially U-shaped) cross section opening at part of the circumferential surface along the axial direction. For another example, the mounting main unit 21 may be a band made of a pair of band-like members, which extend from respective side faces of the housing 11 of the main unit 1 and which are elongated along one direction. In the case where the mounting main unit 21 is a band, the light emitting unit 41 and the light receiving unit 42 of the light emitting/receiving unit 4 are apposed in the housing 11 of the main unit 1.

Various aspects of technologies have been disclosed herein as described above, and main technologies among the disclosure will be summarized below.

A living body information measurement device according to an aspect includes: a mounting part for mounting a finger of a living body; and a light emitting/receiving unit configured to emit measurement light to the finger mounted in the mounting part, and receive detection light based on the measurement light and coming from an inside of the finger for use in obtaining predetermined living body information, wherein the mounting part includes an insertion/removal opening through which the living body finger is to be inserted/removed, a receiving space for receiving the living body finger inserted through the insertion/removal opening, and a mounting main unit having a pad receiving surface facing the receiving space and being configured to receive a pad of the living body finger inserted through the insertion/removal opening, the light emitting/receiving unit is provided to emit measurement light to the receiving space and receive the detection light, and the mounting main unit has a pair of first finger slippage blocking portions at respective side edges of the pad receiving surface in a perpendicular direction perpendicular to an insertion/removal direction in which the living body finger is to be inserted and removed, the first finger slippage blocking portions extending along the insertion/removal direction and being configured to prevent the living body finger received by the pad receiving surface from being shifted in the perpendicular direction.

Such a living body information measurement device has a pair of first finger slippage blocking portions extending in the insertion/removal direction at respective side edges of the pad receiving surface. Thus, the living body finger placed on the pad receiving surface and received by the pad receiving surface can be prevented from turning about the insertion/removal direction or from being shifted in position in the perpendicular direction by the pair of first finger slippage blocking portions. Consequently, such a living body information measurement device is capable of reducing compression applied to a finger and reducing shifting of the position of the finger from the measurement position.

According to another aspect, in the aforementioned living body information measurement device, the pair of first finger slippage blocking portions are one of a pair of projections protruding inward of the receiving space at a predetermined height from the pad receiving surface, a pair of grooves/projections each being a combination of a groove recessed outward of the receiving space with a predetermined depth in the pad receiving surface and a projection provided adjacent to the groove and protruding inward of the receiving space with a predetermined height from the pad receiving surface, and a pair of roughened surfaces obtained through surface roughening.

Such a living body information measurement device can achieve the first finger slippage blocking portions relatively easily with any of projections, grooves/projections, and roughened surfaces. In a case where the pair of first finger slippage blocking portions are constituted by the pair of projections, a living body finger placed on the pad receiving surface and received by the pad receiving surface hits either one of the pair of projections when the living body finger attempts to turn about the insertion/removal direction or shift its position in the perpendicular direction, the turning about the insertion/removal direction and the shift in position in the perpendicular direction are thus blocked, and the finger position is more stabilized. In a case where the pair of first finger slippage blocking portions are constituted by the pair of grooves/projections, a living body finger placed on the pad receiving surface and received by the pad receiving surface hits the projection of at least one of the pair of grooves/projections when the living body finger attempts to turn about the insertion/removal direction or shift its position in the perpendicular direction, the turning about the insertion/removal direction and the shift in position in the perpendicular direction are thus blocked, and the finger position is more stabilized. In addition, the grooves of the grooves/projections allow high breathablitity to be achieved, and the grooves also reduce compression of the finger. In particular, even in cases where the living body information measurement device is worn for a relatively long time for monitoring (checking, observing) the condition of the subject, the finger is held under a better condition. In a case where the pair of first finger slippage blocking portions are constituted by the pair of roughened surfaces, a living body finger placed on the pad receiving surface and received by the pad receiving surface hits either one of the pair of roughened surfaces when the living body finger attempts to turn about the insertion/removal direction or shift its position in the perpendicular direction, the turning about the insertion/removal direction and the shift in position in the perpendicular direction are thus blocked, and the finger position is more stabilized.

According to another aspect, in the living body information measurement device, the device main unit further has: a back receiving surface facing the receiving space and being configured to receive a back of the living body finger inserted through the insertion/removal opening; and a pair of second finger slippage blocking portions at respective side edges of the back receiving surface in the perpendicular direction, the second finger slippage blocking portions extending along the insertion/removal direction and being configured to prevent the living body finger received by the pad receiving surface from being shifted in the perpendicular direction.

In such a living body information measurement device, the pair of second finger slippage blocking portions extending in the insertion/removal direction are provided at respective side edges of the back receiving surface, in addition to the first finger slippage blocking portions at the pad receiving surface. Thus, the living body finger placed on the pad receiving surface and received by the pad receiving surface can be prevented from turning about the insertion/removal direction or from being shifted in position in the perpendicular direction by the respective pairs of first and second finger slippage blocking portions. Consequently, such a living body information measurement device is capable of further reducing compression applied to a finger and further reducing shifting of the position of the finger from the measurement position.

This application is based on Japanese Patent Application No. 2013-231659 filed on November 8, 2013, the content of which is incorporated herein.

While the present invention has been appropriately and fully explained above by way of embodiments with reference to the drawings to describe the present invention, it should be appreciated that those skilled in the art will easily modify and/or improve the above-described embodiments. These modifications or improvements made by those skilled in the art should therefore be understood to be encompassed by the scope of the claims as long as the modifications or improvements depart from the scope of the claims.

### Industrial Applicability

The present invention provides a living body information measurement device for measuring predetermined physiological information from a living body.

## Claims

1. A living body information measurement device comprising:
a mounting part for mounting a finger of a living body; and
a light emitting/receiving unit configured to emit measurement light to the finger mounted in the mounting part, and receive detection light based on the measurement light and coming from an inside of the finger for use in obtaining predetermined living body information, wherein
the mounting part includes an insertion/removal opening through which the living body finger is to be inserted/removed, a receiving space for receiving the living body finger inserted through the insertion/removal opening, and a mounting main unit having a pad receiving surface facing the receiving space and being configured to receive a pad of the living body finger inserted through the insertion/removal opening,
the light emitting/receiving unit is provided to emit measurement light to the receiving space and receive the detection light, and
the mounting main unit has a pair of first finger slippage blocking portions at respective side edges of the pad receiving surface in a perpendicular direction perpendicular to an insertion/removal direction in which the living body finger is to be inserted and removed, the first finger slippage blocking portions extending along the insertion/removal direction and being configured to prevent the living body finger received by the pad receiving surface from being shifted in the perpendicular direction.

2. The living body information measurement device according to claim 1, wherein the pair of first finger slippage blocking portions are one of a pair of projections protruding inward of the receiving space at a predetermined height from the pad receiving surface, a pair of grooves/projections each being a combination of a groove recessed outward of the receiving space with a predetermined depth in the pad receiving surface and a projection provided adjacent to the groove and protruding inward of the receiving space with a predetermined height from the pad receiving surface, and a pair of roughened surfaces obtained through surface roughening.

3. The living body information measurement device according to claim 1 or 2, wherein the device main unit further has:
a back receiving surface facing the receiving space and being configured to receive a back of the living body finger inserted through the insertion/removal opening; and
a pair of second finger slippage blocking portions at respective side edges of the back receiving surface in the perpendicular direction, the second finger slippage blocking portions extending along the insertion/removal direction and being configured to prevent the living body finger received by the pad receiving surface from being shifted in the perpendicular direction.
